(19) Europäisches Patentamt
European Patent Office
Office européen des brevets

(11) Veröffentlichungsnummer: **0 193 013**
**B1**

# (12) EUROPÄISCHE PATENTSCHRIFT

(45) Veröffentlichungstag der Patentschrift: **19.04.89**

(21) Anmeldenummer: **86101638.4**

(22) Anmeldetag: **08.02.86**

(51) Int. Cl.⁴: **C 07 D 401/14**, C 07 D 409/14, C 07 D 403/04, C 07 D 405/14, C 07 D 413/14, C 07 D 403/14, C 07 D 417/14, A 61 K 31/50

(54) **Benzimidazolyl-pyridazinone.**

(30) Priorität: **19.02.85 DE 3505609**

(43) Veröffentlichungstag der Anmeldung: **03.09.86 Patentblatt 86/36**

(45) Bekanntmachung des Hinweises auf die Patenterteilung: **19.04.89 Patentblatt 89/16**

(84) Benannte Vertragsstaaten: **AT BE CH DE FR GB IT LI NL SE**

(56) Entgegenhaltungen:
**EP-A-0 008 391**

**Medicinal Chemistry, A. Burger, Teil I, p. 77**

**Die Akte enthält technische Angaben, die nach dem Eingang der Anmeldung eingereicht wurden und die nicht in dieser Patentschrift enthalten sind.**

(73) Patentinhaber: **MERCK PATENT GESELLSCHAFT MIT BESCHRÄNKTER HAFTUNG, Frankfurter Strasse 250 Postfach 4119, D-6100 Darmstadt (DE)**

(72) Erfinder: **Prücher, Helmut, Köningsbergerstrasse 9, D-6148 Heppenheim (DE)**
Erfinder: **Jonas, Rochus, Dr., Kesselhutweg 12, D-6100 Darmstadt (DE)**
Erfinder: **Piulats, Jaime, Dr., Rvds. Quintin Mallofré 2, Barcelona 30 (ES)**
Erfinder: **Klockow, Michael, Dr., Fuchsenhütte 42, D-6101 Rossdorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 193 013 B1

LIBER, STOCKHOLM 1989

**Beschreibung**

Gegenstand der Erfindung sind neue Benzimidazolyl-pyridazinone der Formel I

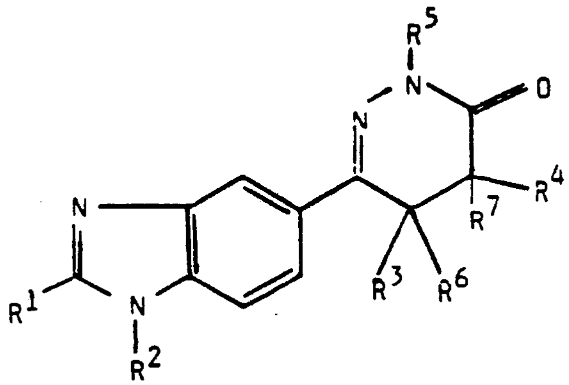

I

worin

$R^1$ einen unsubstituierten oder ein- oder mehrfach durch Alkyl, Alkoxy, Alkylthio, Halogen, OH, SH, Amino, Alkylamino, Dialkylamino, Acylamino mit 1 - 8 C-Atomen, Nitro, COOH, COOAlkyl und/oder CN substituierten Styryl- oder ein- oder zweikernigen, 1-4 Heteroatome enthaltenden Heteroarylrest,

$R^2$, $R^3$, $R^4$ und $R^5$ jeweils H oder Alkyl und

$R^6$ und $R^7$ jeweils H oder zusammen eine C-C-Bindung bedeuten und worin die Alkyl- und Alkoxygruppen jeweils 1 - 4 C-Atome enthalten,

sowie deren Salze.

Ähnliche Verbindungen sind aus der DE-OS-2 837 161 bekannt, die der EP-A-8391 entspricht.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit wert volle pharmakologische Eigenschaften besitzen. Insbesondere zeigen sie eine Wirkung auf die Herzkraft (positiv inotrope Wirksamkeit): ferner wirken die Substanzen vasodilatierend und fördern daher die Durchblutung. Die vasodilatierende Wirkung und die Herzwirkung kann z. B. an narkotisierten oder wachen Hunden, Katzen, Affen oder Minischweinen, die positiv inotrope Wirkung auch an isolierten Herzpräparationen (z. B. Vorhof, Papillarmuskel oder perfundiertes Ganzherz) von Ratte, Meerschweinchen, Katze oder Hund ermittelt werden, z. B. nach Methoden, wie sie in Arzneimittelforschung, Band 31 (I) Nr. 1a (1981), Seiten 141 bis 170, oder von Schliep et al. im 9th International Congress of Pharmacol., London, Abstracts of papers 9P, beschrieben sind.

Weiterhin treten antithrombotische, thrombozytenaggregationshemmende und die Erythrozytenform beeinflussende Eigenschaften auf. Die Beeinflussung der Thrombozytenfunktion im Sinne einer Aggregationshemmung kann an der Ratte ex vivo im Test nach Born (Nature 194, 927 - 929, 1962) nachgewiesen werden. Die antithrombotische Wirkung zeigt sich in der Verlängerung der Blutungszeit nach Stella (Thrombos. Res. 7, 709 - 716, 1975) in der Verminderung des Thrombusgewichtes bei der kälteinduzierten Thrombosierung der Jugular-Vene bei der Ratte nach Meng (Ther. Ber. 47, 69 - 79, 1975) und der Erhöhung der zur vollständigen Thrombosierung notwendigen Laserimpulse an der Mesenterial-Venole der Ratte, entsprechend einer Abwandlung der Methode nach Kovacs (Microvasc. Res. 6, 194 - 201, 1973).

Die günstige Wirkung auf die Erythrozytenverformbarkeit ist im Nucleoporefilter nach Schmid-Schönbein (Pflüger's Archiv 338, 93 - 114, 1973) nachweisbar. Auch lassen sich günstige Effekte auf die Fibrinolyse/Euglobulinlysiszeit nach v. Kaulla (Progr. Chem. Fibrinol, Thrombol. 1, 131 - 149, 1975; ed J.F. Davidson, Raven Press, N.Y.) feststellen.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den Formeln ist Alkyl vorzugsweise unverzweigt, hat vorzugsweise 1 - 3 C-Atome und steht bevorzugt für Methyl, ferner bevorzugt für Ethyl oder Propyl, weiterhin für Isopropyl, Butyl, Isobutyl, sek.-Butyl oder tert.-Butyl.

Alkoxy ist vorzugsweise unverzweigt, hat vorzugsweise 1 - 3 C-Atome und steht bevorzugt für Methoxy, ferner bevorzugt für Ethoxy oder Propoxy, weiterhin für Isopropoxy, Butoxy, Isobutoxy, sek.-Butoxy oder tert.-Butoxy. Alkylthio ist vorzugsweise unverzweigt, hat bevorzugt 1 - 3 C-Atome und steht bevorzugt für Methylthio, ferner bevorzugt für Ethylthio oder Propylthio, weiterhin für Isopropylthio, Butylthio, Isobutylthio, sek.-Butylthio oder tert.-Butylthio.

Halogen ist vorzugsweise F oder Cl, aber auch Br oder J. Alkylamino ist vorzugsweise Methylamino, ferner bevorzugt Ethylamino oder Propylamino, weiterhin Isopropylamino, Butylamino, Isobutylamino, sek.-Butylamino oder tert.-Butylamino. Dialkylamino ist vorzugsweise Dimethylamino, ferner bevorzugt Methylethylamino, Diethylamino oder Dipropylamino, weiterhin z. B. Diisopropylamino, Dibutylamino,

Diisobutylamino, Di-sek.-butylamino oder Di-tert.-butylamino. Acylamino ist bevorzugt Alkanoylamino mit 1 - 8 C-Atomen z. B. Formamido, Acetamido, Propionamido, Butyramido, Isobutyramido, Valeramido, Hexanamido, Heptanamido, Octanamido, ferner bevorzugt Benzamido, substituiertes Benzamido, z. B. o-, m- oder p-Methylbenzamido, o-, m- oder p-Methoxybenzamido, 3,4-Dimethoxybenzamido, o-, m- oder p-Methylthiobenzamido, o-, m- oder p-Fluorbenzamido, o-, m- oder p-Chlorbenzamido, o-, m- oder p-Brombenzamido, o-, m- oder p-Jodbenzamido, o-, m- oder p-Aminobenzamido, o-, m- oder p-Methylaminobenzamido, o-, m- oder p-Dimethylaminobenzamido, o-, m- oder p-Nitrobenzamido, o-, m- oder p-Carboxybenzamido, o-, m- oder p-Cyanbenzamido, weiterhin bevorzugt unsubstituiertes oder substituiertes Picolinamido, Nicotinamido oder Isonicotinamido. COOAlkyl ist vorzugsweise Methoxycarbonyl oder Ethoxycarbonyl, ferner Propoxycarbonyl, Isopropoxycarbonyl, Butoxycarbonyl, Isobutoxycarbonyl, sek.-Butoxycarbonyl oder tert.-Butoxycarbonyl.

Im einzelnen ist $R^1$ bevorzugt unsubstituiertes oder wie angegeben substituiertes Styryl, unsubstituiertes oder wie angegeben substituiertes 1-, 2- oder 3-Pyrryl, 2- oder 3-Furyl, 2- oder 3-Thienyl, 2-, 3- oder 4-Pyridyl, 2-, 4- oder 5-Thiazolyl, 3-, 4- oder 5-Isothiazolyl, 1-, 3-, 4- oder 5-Pyrazolyl, 1-, 2- oder 4(5)-Imidazolyl, 2-, 4- oder 5-Oxazolyl, 3-, 4- oder 5-Isoxazolyl, 1,2,4-Triazol-1-, -3- oder -5-yl, 1,3,4-Thiadiazol-2-yl, 1- oder 5-Tetrazolyl, 3- oder 4-Pyridazinyl, 2-, 4- oder 5-Pyrimidyl, Pyrazinyl, 2-, 3-, 4-, 5-, 6- oder 7-Indolyl, 2-, 3-, 4-, 5-, 6- oder 7-Benzofuryl, 2-, 3-, 4-, 5-, 6- oder 7-Benzothienyl, 2-, 3-, 4-, 5-, 6-, 7 oder 8-Chinolyl, 1-, 3-, 4-, 5-, 6-, 7- oder 8-Isochinolyl, 2-, 4-, 5-, 6- oder 7-Benzthiazolyl, 1-, 2-, 4-, 5-, 6- oder 7-Benzimidazolyl, 2-, 6- oder 8-Purinyl.

Einzelne bevorzugte substituierte Reste $R^1$ sind o-, m- oder p-Methylstyryl, o-, m- oder p-Methoxystyryl, o-, m- oder p-Ethoxystyryl, 2,3-, 2,4-, 2,5-, 2,6-, 3,4- oder 3,5-Dimethoxystyryl, 3,4,5-Trimethoxystyryl, o-, m- oder p-Methylthiostyryl, o-, m- oder p-Ethylthiostyryl, o-, m- oder p-Fluorstyryl, o-, m- oder p-Chlorstyryl, o-, m- oder p-Bromstyryl, o-, m- oder p-Jodstyryl, o-, m- oder p-Hydroxystyryl o-, m- oder p-Mercaptostyryl, o-, m- oder p-Aminostyryl, o-, m- oder p-Methylaminostyryl, o-, m- oder p-Ethylaminostyryl, o-, m- oder p-Dimethylaminostyryl, o-, m- oder p-Diethylaminostyryl, o-, m- oder p-Formamidostyryl, o-, m- oder p-Acetamidostyryl, o-, m- oder p-Benzamidostyryl, o-, m- oder p-Nitrostyryl, o-, m- oder p-Carboxystyryl, o-, m- oder p-Methoxycarbonylstyryl, o-, m- oder p-Ethoxycarbonylstyryl, o-, m- oder p-Cyanstyryl, 1-Methyl-2-pyrryl, 1-Methyl-3-pyrryl, 3-, 4- oder 5-Methyl-2-furyl, 3-, 4-oder 5-Fluor-2-furyl, 3-, 4- oder 5-Chlor-2-furyl, 5-Brom-2-furyl, 5-Nitro-2-furyl, 3-, 4- oder 5-Methyl-2-thienyl, 3-, 4-oder 5-Fluor-2-thienyl, 3-, 4- oder 5-Nitro-2-thienyl, 3-, 4-oder 5-Dimethylamino-2-thienyl, 3-, 4- oder 5-Formamido-2-thienyl, 2-, 4- oder 5-Methyl-3-thienyl, 3-, 4-, 5- oder 6-methyl-2-pyridyl, 3-, 4-, 5- oder 6-Fluor-2-pyridyl, 3-, 4-, 5- oder 6-Chlor-2-pyridyl, 2-, 4-, 5- oder 6-Methyl-3-pyridyl, 2-, 4-, 5- oder 6-Fluor-3-pyridyl, 2-, 4-, 5- oder 6-Chlor-3-pyridyl, 2-, 4-, 5- oder 6-Hydroxy-3-pyridyl-, 2-, 4-, 5- oder 6-Dimethylamino-3-pyridyl, 2-, 4-, 5- oder 6-Formamido-3-pyridyl, 2-, 4-, 5- oder 6-Acetamido-3-pyridyl, 2- oder 3-Methyl-4-pyridyl, 2- oder 3-Fluor-4-pyridyl, 2- oder 3-Chlor-4-pyridyl, 2,6-Dichlor-4-pyridyl, 4- oder 5-Methyl-2-thiazolyl, 5-Nitro-2-thiazolyl, 2- oder 5-Methyl-4-thiazolyl, 2,4-Dimethyl-5-thiazolyl, 4- oder 5-Methyl-3-isothiazolyl, 3- oder 5-Methyl-4-isothiazolyl, 4- oder 5-Methyl-3-pyrazolyl, 4- oder 5-Methyl-2-imidazolyl, 2- oder 5-Methyl-4-imidazolyl, 4- oder 5-Methyl-2-oxazolyl, 2- oder 5-Methyl-4-oxazolyl, 2- oder 4-Methyl-5-oxazolyl, 4- oder 5-Methyl-3-isoxazolyl, 3- oder 5-Methyl-4-isoxazolyl, 3- oder 4-Methyl-5-isoxazolyl, 5-Methyl-1,3,4-thiadiazol-2-yl.

Besonders bevorzugte Reste $R^1$ sind 2-, 3- oder 4-Pyridyl, ferner 2- oder 3-Thienyl, 2- oder 3-Furyl, 1-Methyl-2-pyrryl, Styryl, p-Dimethylaminostyryl oder 3,4-Dimethoxyatyryl.

$R^2$, $R^4$ und $R^5$ aind jeweils bevorzugt H oder $CH_3$; insbesondere sind $R^2$, $R^4$ und $R^5$ bevorzugt H. $R^3$ ist bevorzugt H, $CH_3$ oder $C_2H_5$, insbesondere $CH_3$.

$R^6$ und $R^7$ sind bevorzugt jeweils H.

Gegenstand der Erfindung sind insbesondere die jenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ig ausgedrückt werden, die der Formel I entsprechen und worin die nicht näher bezeichneten Reste die bei der Formel I angegebene Bedeutung haben, worin jedoch

in Ia — $R^1$ 2-, 3- oder 4-Pyridyl, 2- oder 3-Thienyl, 2- oder 3-Furyl, 1-Methyl-2-pyrryl, Styryl, p-Dimethylaminostyryl oder 3,4-Dimethoxystyryl bedeuten;

in Ib — $R^1$ 2-, 3- oder 4-Pyridyl, 2- oder 3-Thienyl, 2- oder 3-Furyl, 1-Methyl-2-pyrryl, Styryl, p-Dimethylaminostyryl oder 3,4-Dimethoxystyryl,
$R^2$, $R^4$ und $R^5$ jeweils H oder $CH_3$ und
$R^3$ H, $CH_3$ oder $C_2H_5$ bedeuten;

in Ic — $R^1$ 2-, 3- oder 4-Pyridyl oder 2- oder 3-Thienyl,
$R^2$, $R^4$ und $R^5$ jeweils H oder $CH_3$ und
$R^3$ H, $CH_3$ oder $C_2H_5$ bedeuten;

in Id — $R^1$ 2-, 3- oder 4-Pyridyl oder 2- oder 3-Thienyl,
$R^2$, $R^4$ und $R^5$ jeweils H oder $CH_3$
$R^3$ H, $CH_3$ oder $C_2H_5$ und
$R^6$ und $R^7$ jeweils H bedeuten;

in Ie — $R^1$ 2-, 3- oder 4-Pyridyl oder 2- oder 3-Thienyl,
$R^2$, $R^4$ und $R^5$ jeweils H oder $CH_3$ und
$R^3$ H, $CH_3$ oder $C_2H_5$ und

$R^6$ und $R^7$ zusammen eine C-C-Bindung bedeuten;

in If

$R^1$ 2-,3- oder 4-Pyridyl, 2- oder 3-Thienyl, 2- oder 3-Furyl, 1-Methyl-2-pyrryl, Styryl, p-Dimethylaminostyryl oder 3,4-Dimethoxystyryl,
$R^2$ H oder $CH_3$,
$R^3$ $CH_3$ oder $C_2H_5$ und
$R^4$, $R^5$, $R^6$ und $R^7$ jeweils H bedeuten;

in Ig $R^1$ 2-, 3- oder 4-Pyridyl oder 2- oder 3-Thienyl,
$R^2$ H oder $CH_3$,
$R^3$ $CH_3$ oder $C_2H_5$ und
$R^4$, $R^5$, $R^6$ und $R^7$ jeweils H bedeuten.

Besonders bevorzugte Einzelverbindungen der Formel I sind:
a) 5-Methyl-6-[2-(4-pyridyl)-5-benzimidazolyl]-4,5-dihydro-pyridazin-3-on;
b) 5-Methyl-6-[2-(3-pyridyl)-5-benzimidazolyl]-4,5-dihydro-pyridazin-3-on;
c) 5-Methyl-6-[2-(2-pyridyl)-5-benzimidazolyl]-4,5-dihydro-pyridazin-3-on;
d) 5-Methyl-6-[2-(3-thienyl)-5-benzimidazolyl]-4,5-dihydro-pyridazin-3-on;
e) 5-Methyl-6-[1-methyl-2-(4-pyridyl)-5-benzimidazolyl]-4,5-dihydro-pyridazin-3-on sowie
f) 5-Methyl-6-[2-(3-pyrazolyl)-5-benzimidazolyl]-4,5-dihydro-pyridazin-3-on.

Die Verbindungen der Formel I werden im übrigen nach an sich bekannten Methoden hergestellt, wie sie in der Literatur (z. B. in den Standardwerken wie Houben-Weyl, Methoden der Organischen Chemie, Georg-Thieme-Verlag, Stuttgart; inabesondere aber in der DE-OS-2 837 161) beschrieben sind, und zwar unter Reaktionsbedingungen, die für die genannten Umsetzungen bekannt und geeignet sind. Dabei kann man auch von an aich bekannten, hier nicht näher erwähnten Varianten Gebrauch machen.

Die Ausgangsstoffe können gewünschtenfalls auch in situ gebildet verden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

So können die Verbindungen der Formel I erhalten werden durch Reaktion von Diaminen der Formel II mit Säuren der Formel $R^1$-COOH oder ihren reaktionsfähigen Derivaten. Als reaktionsfähige Derivate der Säuren eignen sich insbesondere die entsprechenden Nitrile, Säurehalogenide, Ester, Amide, Imidsäureester, Imidsäurethioester, Imidsäurehalogenide, Amidine, Thiocarbonsäureester, Dithiocarbonsäureester oder Orthoester.

Die Ausgangsstoffe der Formeln II und $R^1$-COOH sind teilweise bekannt. Sofern sie nicht bekannt sind, können sie nach an sich, z. B. aus der DE-OS-2 837 161, bekannten Methoden hergestellt werden. Die Carbonsäuen der Formel $R^1$-COOH sind beispielsweise duch Oxydation entsprechender Aldehyde der Formel $R^1$-CHO zugänglich.

Im einzelnen erfolgt die Umsetzung der Diamine der Formel II mit den Säuren der Formel $R^1$-COOH bzw. ihren reaktionsfähigen Derivaten in Gegenwart oder Abwesenheit eines inerten Lösungmittels bei Temperaturen zwischen etwa -20 und etwa 250°, vorzugsweise zwischen 60 und 150°. Als Lösungsmittel eignen sich beispielsweise Kohlenwasserstoffe wie Benzol, Toluol, Xylole oder Mesitylen; halogenierte Kohlenwasserstoffe wie Dichlormethan, Trichlorethylen oder Chlorbenzol; tertiäre Basen wie Triethylamin, Pyridin oder Picoline; Alkohole wie Methanol, Ethanol oder Isopropanol; Glykole und Glykolether wie Ethylenglykol, Diethylenglykol, 2-Methoxyethanol; Ketone wie Aceton; Ether wie Tetrahydrofuran (THF) oder Dioxan; Amide wie Dimethylformamid (DMF), Sulfoxide wie Dimethylsulfoxid. Auch Gemische dieser Lösungsmittel sind geeignet. In manchen Fällen empfiehlt sich der Zusatz von katalytischen Mengen einer Säure wie p-Toluolsulfonsäure oder der Zusatz eines Dehydratisierungsmittels wie Carbonyldiimidazol, Phosphoroxychlorid, Polyphosphorsäure oder Thionylchlorid, wobei das Dehydratisierungsmittel auch als Lösungsmittel dienen kann.

Verwendet man die freien Carbonsäuren der Formel $R^1$-COOH, so wird die Reaktion zweckmäßig in Gegenwart eines der genannten Dehydratisierungsmittel und gegebenenfalls einer tertiären Base wie Pyridin oder Triethylamin durchgeführt, vorzugsweise bei Temperaturen zwischen -20 und 150°. Eine besonders günstige Methode besteht in der Umsetzung des Diamins mit der Säure in THF in Gegenwart von Carbonyldiimidazol bei Raumtemperatur.

Die Umsetzung kann auch stufenweise durchgeführt werden. So ist es z. B. möglich, II mit einem Säurechlorid der Formel $R^1$-COCl partiell zu einem 6-(3-$R^1$CO-NH-4-$R^2$NH-phenyl)-pyridazin-3-on oder einem 6-(3-Amino-4-$R^1$CO-N$R^2$-phenyl)-pyridazin-3-on (oder den entsprechenden 4,5-Dihydropyridazin-3-onen; oder zu Gemischen der Isomeren) zu acylieren, das anschließend, z. B. durch Kochen mit Essigsäure, zu I dehydratisiert wird.

Es ist auch möglich, an Stelle der Säure einen entsprechenden Aldehyd der Formel $R^1$-CHO zu verwenden, wenn man gleichzeitig in Gegenwart eines Oxydationsmittels arbeitet. Bevorzugt verwendet man als Oxydationsmittel Schwefel in einem Kohlenwasserstoff wie Benzol, Toluol, Xylol oder Mesitylen oder Natriumdisulfit in Lösungsmitteln wie Dimethylacetamid, jeweils bei Temperaturen zwischen etwa 80 und etwa 200°. Die Aldehyde sind in der Regel bekannt und beispielsweise durch Formylierung der entsprechenden Grundverbindungen $R^1$-H erhältlich, z. B. nach der Methode von Vilsmeier-Haack.

Die Verbindungen der Formel I können auch durch Cyclisierung von Verbindungen der Formel III erhalten werden. In diesen bedeuten die Reste $Z^1$ und $Z^2$ vorzugsweise zusammen 0.

Die Cyclisierung wird zweckmäßigerweise in einem Lösungsmittel wie Ethanol, Isopropanol, Essigsäure, Chlorbenzol, Ethylenglykol, DMF, Tetralin oder in einem Überschuß des zur Herstellung der Verbindung der allgemeinen Formel II verwendeten Acylierungsmittels, z. B. in $R^1CN$, $(R^1CO)_2O$, $R^1COOH$, $R^1CSOH$ oder $R^1CSSH$ oder deren Estern, Amiden oder Halogeniden, bei erhöhten Temperaturen, z. B. bei Temperaturen zwischen 0 und 250°, gegebenenfalls in Gegenwart eines Kondensationsmittels wie Phosphoroxychlorid, Thionylchlorid, Sulfurylchlorid, Schwefelsäure, Salzsäure, Phosphorsäure, Essigsäure, Essigsäureanhydrid oder gegebenenfalls auch in Gegenwart einer Base wie Kalium-ethylat oder Kalium-tert.-butylat durchgeführt. Die Cyclisierung kann jedoch auch ohne Lösungsmittel und/oder ohne Kondensationsmittel durchgeführt werden.

Besonders vorteilhaft wird die Umsetzung jedoch in der Weise durchgeführt, daß ein entsprechendes 6-(Acylamino-nitro-phenyl)-pyridazin-3-on durch Reduktion, beispielsweise durch Reduktion mit Wasserstoff in Gegenwart eines Hydrierungskatalysators wie Raney-Nickel, Platin oder Palladium/Kohle, durch Reduktion mit Metallen wie Eisen, Zinn oder Zink oder durch Reduktion mit Metallsalzen wie Eisen-(II)-sulfat, Zinn-(II)-chlorid oder Chrom-(II)-chlorid, in eine entsprechende Verbindung der Formel III übergeführt wird, welche gegebenenfalls im gleichen Reaktionsgemisch erforderlichenfalls in Gegenwart einer Säure wie Salzsäure, Schwefelsäure, Phosphorsäure, Essigsäure oder einer Carbonsäure der Formel $R^1COOH$, oder in Gegenwart eines Kondensationsmittels wie Phosphoroxychlorid oder in Gegenwart einer Base wie Kalium-ethylat gegebenenfalls in einem Lösungsmittel wie Ethanol, Isopropanol, Ethylenglykol, DMF, Dimethylsulfoxid oder Chlorbenzol bei Temperaturen zwischen 0 und 250° cyclisiert wird.

Die Verbindungen der Formel I sind weiterhin erhältlich durch Reaktion einer Ketosäure der Formel IV oder eines ihrer reaktionsfähigen Derivate mit einem Hydrazin der Formel $R^5$-NH-$NH_2$ oder einem seiner reaktionsfähigen Derivate.

Die Carbonsäuren der Formel IV können nach an sich bekannten Methoden hergestellt werden, beispielsweise analog DE-OS-2 837 161.

Als reaktionsfähige Derivate der Carbonsäuren der Formel IV eignen sich insbesondere die Ester, z. B. die Alkylester; worin die Alkylgruppe vorzugsweise 1 - 4 C-Atome besitzt, insbesondere die Methyl- und Ethylester, ferner die Nitrile, die Säurehalogenide, z. B. Säurechloride oder Säurebromide, und die Amide. Weitere geeignete reaktionsfähige Derivate der Carbonsäuren der Formel IV können während der Reaktion in situ gebildet werden, ohne daß man sie isoliert. Dazu gehören beispielsweise die Hydrazone der Formel Ben-C($=NNHR^5$)-$CR^3R^6=CR^4R^7$-COOH und die Hydrazide der Formel Ben-CO-$CR^3R^6$-$CR^4R^7$-CO-$NHNHR^5$ (worin Ben den 1-$R^2$-2-$R^1$-5-benzimidazolylrest bedeutet).

Als reaktionsfähige Derivate des Hydrazins der Formel $R^5$-NH-$NH_2$ eignen sich z. B. die entsprechenden Hydrazinhydrate, Acethydrazide, Semicarbazide oder Carbazinsäureester.

Für die Umsetzung mit den Carbonsäuren der Formel IV bzw. ihren reaktionsfähigen Derivaten verwendet man vorteilhaft 1 - 5 Äquivalente des Hydrazins bzw. reaktionsfähigen Hydrazinderivats, das gleichzeitig als Lösungsmittel dienen kann. Zweckmäßiger ist es jedoch, ein zusätzliches inertes Lösungsmittel zuzusetzen. Als inerte Lösungsmittel eignen sich vorzugsweise Alkohole wie Methanol, Ethanol, Isopropanol, n-Butanol, Isoamylalkohol, Glykole und deren Ether wie Ethylenglykol, Diethylenglykol, Ethylenglykolmonomethyl- oder -monoethylether (Methylglykol oder Ethylglykol), Carbonsäuren wie Ameisen-, Essig- oder Propionsäure, ferner Ether, insbesondere wasserlösliche Ether wie Tetrahydrofuran, Dioxan oder Ethylenglykoldimethylether (Diglyme); ferner Wasser sowie Gemische dieser Lösungsmittel untereinander, insbesondere Gemische mit Wasser, z. B. wässeriges Ethanol. Man kann auch eine Säure wie Schwefelsäure oder p-Toluolsulfonsäure als Katalysator zusetzen. Die Reaktionstemperaturen liegen zweckmäßig zwischen etwa 0 und 200°, vorzugsweise zwischen 20 und 100°, die Reaktionszeiten zwischen etwa 1 und 48 Stunden.

Ein Tetrahydropyridazinon der Formel I ($R^6=R^7=H$) kann gewünschtenfalls zu einem entsprechenden Dihydropyridazinon (I, $R^6$ und $R^7$ bedeuten zusammen eine C-C-Bindung) dehydriert werden. Als Dehydrierungsmittel eignen sich z. B. Brom, $PCl_5$, 3-Nitrobenzolsulfonsäure-Na-Salz, $CrO_3$, N-Bromsuccinimid, $H_2O_2$, 2,3-Dichlor-5,6-dicyan-1,4-benzochinon (DDQ) oder $NaNO_2$ in einem Lösungsmittel wie Dioxan, Essigsäure, Propionsäure oder Nitrobenzol bei Temperaturen zwischen etwa 0 und 120°, vorzugsweise zwischen 50 und 100°.

Man kann ferner in an sich bekannter Weise in einer Verbindung der Formel I eine funktionelle Gruppe in eine andere funktionelle Gruppe umwandeln.

So kann man z. B. OH-, SH- oder NH-Gruppen zu Alkoxy-, Alkylthio-, Monoalkylamino- oder Dialkylaminogruppen alkylieren, NH-Gruppen zu Acylaminogruppen acylieren, $NO_2$-Gruppen zu $NH_2$-Gruppen reduzieren, Carboxylgruppen zu COOAlkylgruppen verestern oder über die entsprechenden Amide in CN-Gruppen überführen, Estergruppen zu COOH-Gruppen verseifen, Halogenatome mit Metallcyaniden in CN-Gruppen umwandeln. Alle diese Umwandlungen gelingen nach Methoden, die in der Literatur (z. B. Houben-Weyl, l.c.) beschrieben und dem Fachmann geläufig sind.

Eine Base der Formel I kann mit einer Säure in das zugehörige Säureadditionssalz überführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische,

araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z. B. Pikrate, können zur Aufreinigung der Verbindungen der Formel I verwendet werden.

Eine Säure der Formel I kann durch Umsetzung mit einer Base in eines ihrer Metall- bzw. Ammoniumsalze übergeführt werden; insbesondere kommen dafür die Na-, K-, Mg- Ca- und Ammoniumsalze in Betracht, ferner substituierte Ammoniumsalze, z. B. die Dimethyl-, Diethyl-, Monoethanol-, Diethanol-, Triethanol-, Cyclohexyl- und Dicyclohexylammoniumsalze, weiterhin Dibenzylethylendiammoniumsalze oder Salze mit N-Methyl-D-glucamin oder mit basischen Aminosäuren wie Arginin oder Lysin.

Die freien Basen der Formel I können, falls erwünscht, aus ihren Salzen durch Behandlung mit starken Basen wie Natrium- oder Kaliumhydroxid, Natrium- oder Kaliumcarbonat in Freiheit gesetzt werden.

Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch-aktiven Trennmittel Diastereomere gebildet. Als Trennmittel eignen sich z. B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie β-Camphersulfonsäure.

Natürlich ist es auch möglich, optisch aktive Verbindungen der Formel I nach den oben beschriebenen Methoden zu erhalten, indem man Ausgangsstoffe verwendet, die bereits optisch aktiv sind.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei konnen sie zusammen mit mindestens einem festen, flüssigen und/oder halbflüssigen Trager- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenteralb oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur Parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen zubereitungen können sterilisiert sein und/oder Hilfsstoffe wie Gleit-, Konservierungs-, Stabilisierungsund/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I können bei der Bekämpfung von Krankheiten, insbesondere von Herzinsuffizienz, verwendet werden.

Dabei werden die erfindungsgemäßen Substanzen in der Regel in Analogie zu bekannten positiv inotrop wirksamen Substanzen wie Amrinon verabreicht, vorzugsweise in Dosierungen zwischen etwa 1 und 100 mg insbesondere zwischen 2 und 20 mg pro Dosierungseinheit. Die tägliche Dosierung liegt vorzugsweise zwischen etwa 0,02 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

In den nachfolgenden Beispielen bedeutet "übliche Aufarbeitung":

Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie und/oder Kristallisation.

Vor- und nachstehend sind alle Temperaturen in Celsiusgraden angegeben.

**Beispiel 1**

Ein Gemisch von 17 g Isonicotinoylchlorid, 21,8 g 5-Methyl-6-(3,4-diaminophenyl)-4,5-dihydro-pyridazin-3-on ("IIa"; F. 195 - 196°) und 400 ml Chlorbenzol wird 2 Stunden gekocht. Nach dem Abkühlen arbeitet man wie üblich auf und erhält zunächst ein öliges Gemisch aus 5-Methyl-6-(3-amino-4-isonicotinamidophenyl)- und 5-Methyl-6-(3-isonicotinamido-4-aminophenyl)-4,5-dihydro-pyridazin-3-on, das in 400 ml Essigsäure gelöst und 3 Stunden gekocht wird. Nach dem Abkühlen erhält man 5-Methyl-6-[2-(4-pyridyl)-5-benzimidazoly]-4,5-dihydro-pyridazin-3-on ("M"), Dihydrat, F. 195 - 198° (Zers.). Dihydrochlorid-dihydrat, F. 224 - 227°. Methansulfonat, F. 282 - 285°.

Analog erhält man mit den entsprechenden Säurechloriden die folgenden 5-Methyl-6-(2-$R^1$-5-benzimidazolyl)-4,5-dihydropyridazin-3-one:

$R^1$ = Styryl, Hydrochlorid, Zers. ab 306°
p-Methylstyryl
o-Methoxystyryl
m-Methoxystyryl
p-Methoxystyryl, Hydrochlorid, Monohydrat, Zers. ab 306°.
3,4-Dimethoxystyryl, Hydrochlorid, Zers. ab 285°
3,4,5-Trimethoxystyryl
p-Methylthiostyryl
o-Fluorstyryl
m-Fluorstyryl
p-Fluorstyryl
o-Chlorstyryl
m-Chlorstyryl
p-Chlorstyryl
p-Bromstyryl
p-Jodstyryl
p-Hydroxystyryl
p-Mercaptostyryl
p-Aminostyryl
p-Methylaminostyryl
o-Dimethylaminostyryl
m-Dimethylaminostyryl
p-Dimethylaminostyryl, Hemihydrat, Zers. ab 268°
p-Acetamidostyryl
p-Nitrostyryl
p-Carboxystyryl
p-Methoxycarbonylstyryl
p-Ethoxycarbonylstyryl
p-Cyanstyryl
2-Pyrryl
1-Methyl-2-pyrryl, Hydrochlorid, Zers. ab 324°
2-Furyl, Hydrochlorid, Zers. ab 276°
5-Methyl-2-furyl
5-Brom-2-furyl
5-Nitro-2-furyl
3-Furyl
2-Thienyl, Hydrochlorid, Zers. ab 316°
5-Methyl-2-thienyl
5-Methoxy-2-thienyl, Hydrochlorid-hemihydrat, Zers. ab 220°
5-Chlor-2-thienyl, F. 310 - 313°
4-Brom-2-thienyl, Hydrochlorid-monohydrat, F. 304 - 306°
5-Brom-2-thienyl, Hydrochlorid, F. 313 - 315°
5-Nitro-2-thienyl
5-Cyan-2-thienyl, Hydrochlorid-hemihydrat, Zers. ab 303°
3-Thienyl, Hydrochlorid, F. >300°
2-Pyridyl, Hydrochlorid-monohydrat, Zers. ab 222°
6-Methyl-2-pyridyl, Dihydrochlorid-hemihydrat, Zers. ab 312°
3-Pyridyl, Dihydrat, Zers. ab 173°
4-Chlor-3-pyridyl
2,6-Dichlor-4-pyridyl
4-Thiazolyl
4-Methyl-2-thiazolyl
2,4-Dimethyl-5-thiazolyl

3-Pyrazolyl, Hydrochlorid-hemihydrat, kein F. bis 320°
5-Methyl-3-pyrazolyl
2-Imidazolyl
4(5)-Imidazolyl
2-Methyl-4(5)-imidazolyl
5-Methyl-4-imidazolyl Dihydrochlorid-monohydrat, kein F. bis 320°
3-Isoxazolyl
5-Methyl-3-isoxazolyl
1,2,4-Triazol-5-yl
Pyrazinyl
4-Indolyl, Hydrochlorid-hydrat, Zers. ab 338°
5-Indolyl
2-Chinolyl
4-Chinolyl
1-Isochinolyl.

**Beispiel 2**

Ein Gemisch von 12,3 g Isonicotinsäure, 23,2 g 5-Methyl-6-(3-amino-4-methylaminophenyl)-4,5-dihydro-pyridazin-3-on, 16,2 g N,N'-Carbonyldiimidazol und 600 ml THF wird 16 Stunden bei 25° gerührt. Nach üblicher Aufarbeitung erhält man ein öliges Gemisch aus 5-Methyl-6-(3-amino-4-N-methylisonicotinamidophenyl)- und 5-Methyl-6-(3-isonicotinamido-4-methylaminophenyl)-4,5-dihydropyridazin-3-on, das in 400 ml Essigsäure gelöst wird. Nach 3-std. Kochen der Lösung, Abkühlen und üblicher Aufarbeitung erhält man 5-Methyl-6-[1-methyl-2-(4-pyridyl)-5-benzimidazoly]-4,5-dihydro-pyridazin-3-on; Dihydrochlorid, Zers. ab 298°.

Analog erhält man mit den entsprechenden Säuren die folgenden 5-Methyl-6-(1-methyl-2-$R^1$-5-benzimidazolyl)-4,5-dihydro-pyridazin-3-one:

$R^1$ = Styryl
p-Methylstyryl
p-Methoxystyryl
3,4-Dimethoxystyryl
p-Fluorstyryl
p-Chlorstyryl
p-Dimethylaminostyryl
2-Pyrryl
1-Methyl-2-pyrryl
2-Furyl
5-Methyl-2-furyl
3-Furyl
2-Thienyl
3-Thienyl
2-Pyridyl, Hydrochlorid-dihydrat, Zers. ab 245°
6-Methyl-2-pyridyl
3-Pyridyl, Dihydrochlorid-hydrat, Zers. ab 222°
4-Chlor-3-pyridyl
2,6-Dichlor-4-pyridyl
4-Thiazolyl
4-Methyl-2-thiazolyl
2,4-Dimethyl-5-thiazolyl
3-Pyrazolyl
5-Methyl-3-pyrazolyl
2-Imidazolyl
4(5)-Imidazolyl
2-Methyl-4(5)-imidazolyl
1,2,4-Triazol-5-yl
4-Chinolyl.

Aus 5-Methyl-6-(3-amino-4-ethylaminophenyl)-4,5-dihydro-pyridazin-3-on erhält man analog die entsprechenden 5-Methyl-6-(1-ethyl-2-$R^1$-5-benzimidazolyl)-4,5-dihydro-pyridazin-3-one, z. B. die mit

$R^1$ = Styryl
2-Thienyl, F. 199 - 200°
2-Pyridyl, F. 233 - 234°
3-Pyridyl, F. 204 - 206°
4-Pyridyl, F. 229 - 230°.

**Beispiel 3**

Ein Gemisch von 10,7 g Pyridin-4-aldehyd 21,8 g IIa 19 g Natriumdisulfit ($Na_2S_2O_5$) und 250 ml Ethanol wird 6 Stunden gekocht. Man dampft ein, arbeitet wie üblich auf und erhält "M", Dihydrat, F. 195 - 198° (Zers.).

Analog erhält man mit 5-Ethyl-6-(3,4-diaminophenyl)-4,5-dihydro-pyridazin-3-on das 5-Ethyl-6-[2-(4-pyridyl)-5-benzimidazoly]-4,5-dihydropyridazin-3-on (Dihydrochlorid-dihydrat, F. 299 - 302°) sowie die folgenden 5-Ethyl-6-(2-$R^1$-5-benzimidazolyl)-4,5-dihydropyridazin-3-one:

$R^1$ = Styryl
p-Methylstyryl
p-Methoxystyryl
3,4-Dimethoxystyryl
p-Fluorstyryl
p-Chlorstyryl
p-Dimethylaminostyryl
2-Pyrryl
1-Methyl-2-pyrryl
2-Furyl
5-Methyl-2-furyl
3-Furyl
2-Thienyl
3-Thienyl
2-Pyridyl
6-Methyl-2-pyridyl
3-Pyridyl
4-Chlor-3-pyridyl
2,6-Dichlor-4-pyridyl
4-Thiazolyl
4-Methyl-2-thiazolyl
2,4-Dimethyl-5-thiazolyl
3-Pyrazolyl
5-Methyl-3-pyrazolyl
2-Imidazolyl
4(5)-Imidazolyl
2-Methyl-4(5)-imidazolyl
1,2,4-Triazol-5-yl
4-Chinolyl.

Analog erhält man mit 6-(3,4-Diaminophenyl)-4,5-dihydro-pyridazin-3-on das 6-[2-(4-Pyridyl)-5-benzimidazoly]-4,5-dihydro-pyridazin-3-on (Hydrochlorid, F. >320°) sowie die folgenden 6-(2-$R^1$-5-benzimidazolyl)-4,5-dihydro-pyridazin-3-one:

$R^1$ = Styryl
p-Methylstyryl
p-Methoxystyryl
3,4-Dimethoxystyryl
p-Fluorstyryl
p-Chlorstyryl
p-Dimethylaminostyryl
2-Pyrryl
1-Methyl-2-pyrryl
2-Furyl
5-Methyl-2-furyl
3-Furyl
2-Thienyl
3-Thienyl
2-Pyridyl
6-Methyl-2-pyridyl
3-Pyridyl
4-Chlor-3-pyridyl
2,6-Dichlor-4-pyridyl
4-Thiazolyl
4-Methyl-2-thiazolyl
2,4-Dimethyl-5-thiazolyl

3-Pyrazolyl
5-Methyl-3-pyrazolyl
2-Imidazolyl
4(5)-Imidazolyl
2-Methyl-4(5)-imidazolyl
1,2,4-Triazol-5-yl
4-Chinolyl.

**Beispiel 4**

Eine Lösung von 1 g 5-Methyl-6-(3-amino-4-isonicotinamido-phenyl)-4,5-dihydro-pyridazin-3-on [erhältlich durch Reaktion von 3-(3-Nitro-4-aminobenzoyl)-buttersäuremethylester mit Isonicotinoylchlorid zu 3-(3-Nitro-4-isonicotinamidobenzoyl)-buttersäuremethylester, Umsetzung mit Hydrazinhydrat zu 5-Methyl-6-(3-nitro-4-isonicotinamidophenyl)-4,5-dihydro-pyridazin-3-on und Hydrierung an Pd-C] in 10 ml Essigsäure wird 1,5 Stunden gekocht, eingedampft und wie üblich aufgearbeitet. Man erhält "M", Dihydrat, F. 195 - 198° (Zers.).

Analog sind die übrigen in Beispiel 1 - 3 genannten Verbindungen erhältlich.

**Beispiel 5**

In eine siedende Suspension von 1 g 5-Methyl-6-(3-amino-4-nicotinamidophenyl)-4,5-dihydro-pyridazin-3-on in 50 ml Isopropanol wird 2 Stunden lang HCl-Gas eingeleitet. Nach Eindampfen und üblicher Aufarbeitung erhält man 5-Methyl-6-[2-(3-pyridyl)-5-benzimidazoly]-4,5-dihydro-pyridazin-3-on, Dihydrat, Zers. ab 173°.

Analog erhält man aus 4-Methyl-6-(3-amino-4-isonicotinamidophenyl)-4,5-dihydro-pyridazin-3-on das 4-Methyl-6-[2-(4-pyridyl)-5-benzimidazoly]-4,5-dihydro-pyridazin-3-on.

**Beispiel 6**

Man rührt ein Gemisch von 3,1 g 3-Methyl-4-oxo-4-[2-(4-pyridyl)-5-benzimidazoly]-buttersäure [erhältlich aus 2-(4-Pyridyl)-benzimidazol und Methyl-succinanhydrid/$AlCl_3$] und 2 g Hydrazinhydrat in 70 ml Essigsäure 2 Stunden bei 100°, arbeitet wie üblich auf und erhält "M", Dihydrat, F. 195 - 198° (Zers.)

Analog erhält man mit den entsprechenden Alkylhydrazinen:

1,5-Dimethyl-6-[2-(4-pyridyl)-5-benzimidazoly]-4,5-dihydro-pyridazin-3-on, Dihydrochlorid-dihydrat, F. 228 - 235°

1-Ethyl-5-methyl-6-[2-(4-pyridyl)-5-benzimidazoly]-4,5-dihydropyridazin-3-on, Dimethansulfonat, F. 232 - 233°

1-Propyl-5-methyl-6-[2-(4-pyridyl)-5-benzimidazoly]-4,5-dihydropyridazin-3-on

1-Isobutyl-5-methyl-6-[2-(4-pyridyl)-5-benzimidazolyl]-4,5-dihydropyridazin-3-on

1,5-Diethyl-6-[2-(4-pyridyl)-5-benzimidazoly]-4,5-dihydropyridazin-3-on.

**Beispiel 7**

Eine Lösung von 3,42 g "M"-Hydrochlorid in 100 ml Essigsäure wird unter Rühren bei 70° tropfenweise mit einer Lösung von 1,2 ml Brom in 12 ml Essigsäure versetzt. Man hält noch 1 Stunde bei 70°, dampft ein, arbeitet wie üblich auf und erhält 5-Methyl-6-[2-(4-pyridyl)-5-benzimidazoly]-pyridazin-3-on. Kein F. bis 330°.

Analog erhält man aus den entsprechenden 4,5-Dihydropyridazin-3-onen die nachfolgenden 5-Methyl-6-(2-$R^1$-5-benzimidazolyl)-pyridazin-3-one:

$R^1$ = Styryl
p-Methylstyryl
p-Methoxystyryl
3,4-Dimethoxystyryl
p-Fluorstyryl
p-Chlorstyryl
p-Dimethylaminostyryl

2-Pyrryl
1-Methyl-2-pyrryl
2-Furyl
5-Methyl-2-furyl
3-Furyl
2-Thienyl
3-Thienyl
2-Pyridyl
6-Methyl-2-pyridyl
3-Pyridyl
4-Chlor-3-pyridyl
2,6-Dichlor-4-pyridyl
4-Thiazolyl
4-Methyl-2-thiazolyl
2,4-Dimethyl-5-thiazolyl
3-Pyrazolyl
5-Methyl-3-pyrazolyl
2-Imidazolyl
4(5)-Imidazolyl
2-Methyl-4(5)-imidazolyl
1,2,4-Triazol-5-yl
4-Chinolyl

sowie die entsprechenden 6-(2-$R^1$-5-benzimidazolyl)-pyridazinone, ihre 5-Ethylderivate sowie die entsprechenden 5-Methyl-6-(1-methyl-2-$R^1$-5-benzimidazolyl)-pyridazinone, ferner:

4-Methyl-6-[2-(4-pyridyl)-5-benzimidazoly]-pyridazin-3-on
1,5-Dimethyl-6-[2-(4-pyridyl)-5-benzimidazoly]-pyridazin-3-on
1,5-Diethyl-6-[2-(4-pyridyl)-5-benzimidazoly]-pyridazin-3-on.

**Beispiel 8**

Ein Gemisch von 3,05 g "M", 2,5 g 2,3-Dichlor-5,6-dicyan-1,4-benzochinon (DDQ) und 25 ml Dioxan wird 10 Stunden unter $N_2$ gekocht. Man dampft ein, arbeitet wie üblich auf und erhält 5-Methyl-6-[2-(4-pyridyl)-5-benzimidazoly]-pyridazin-3-on.

**Beispiel 9**

Eine Lösung von 1 g 5-Methyl-6-[2-(5-nitro-2-furyl)-5-benzimidazoly]-4,5-dihydro-pyridazin-3-on in 30 ml Methanol wird an 0,2 g 5 %-igem Pd-C bei 20° und 1 bar bis zum Stillstand hydriert. Man filtriert, dampft ein und erhält 5-Methyl-6-[2-(5-amino-2-furyl)-5-benzimidazoly]-4,5-dihydro-pyridazin-3-on.

**Beispiel 10**

Zu einer Lösung von 309 mg 5-Methyl-6-[2-(5-amino-2-furyl)-5-benzimidazoly]-4,5-dihydro-pyridazin-3-on und 0,2 ml Triethylamin in 10 ml Chloroform gibt man 0,3 ml Acetylchlorid in 3 ml Chloroform und kocht 3 Stunden. Man arbeitet wie üblich auf und erhält 5-Methyl-6-[2-(5-acetamido-2-furyl)-5-benzimidazoly]-4,5-dihydro-pyridazin-3-on.

**Beispiel 11**

Ein Gemisch aus 1 g 5-Methyl-6-(2-p-methoxycarbonylstyryl-5-benzimidazolyl)-4,5-dihydro-pyridazin-3-on und 60 ml 10 %-iger Natronlauge wird 12 Stunden bei 20° gerührt. Nach Ansäuern mit HCl und üblicher Aufarbeitung erhält man 5-Methyl-6-(2-p-carboxystyryl-5-benzimidazolyl)-4,5-dihydro-pyridazin-3-on-hydrochlorid.

**Beispiel 12**

Ein Gemisch von 10,7 g Pyridin-4-aldehyd, 21,8 g IIa, 15,4 g Benzyliden-malodinitril und 500 ml Ethanol wird 3 Stunden gekocht. Nach üblicher Aufarbeitung erhält man "M", Dihydrat, F. 195 - 198° (Zers.).

Analog erhält man mit (+)-IIa [herstellbar durch Reaktion von (-)-3-(4-Chlor-3-nitrobenzoyl)-buttersäure mit Hydrazin zu (+)-5-Methyl-6-(4-chlor-3-nitrophenyl)-4,5-dihydro-pyridazin-3-on, Umsetzung mit Benzylamin zu (+)-5-Methyl-6-(4-benzylamino-3-nitro-phenyl)-4,5-dihydro-pyridazin-3-on und Hydrierung in methanolischer Salzsäure] das (+)-"M", F. 216 - 220°. Methansulfonat, F. 280 - 285°; $[\alpha]_D^{20}$ + 238,0° (in Wasser).

Analog erhält man mit (-)-IIa das (-)-"M", F. 219 - 223°. Methansulfonat, F. 280 - 285°; $[\alpha]_D^{20}$ - 237,9° (in Wasser).

Die nachstehenden Beispiele betreffen pharmazeutische Zubereitungen, die Verbindungen der Formel I oder ihre Säureadditionssalze enthalten:

**Beispiel A:** Tabletten

Ein Gemisch von 1 kg "M", 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

**Beispiel B:** Dragees

Analog Beispiel A werden Tabletten gepreßt, die anschließend in üblicher Weise mit einem Überzug aus Saccharose, Kartoffelstärke, Talk,Tragant und Farbstoff überzogen werden.

**Beispiel C:** Kapseln

1 kg 5-Methyl-6-[2-(3-thienyl)-5-benzimidazoly]-4,5-dihydro-pyridazin-3-on-hydrochlorid wird in üblicher Weise in Hartgelatinekapseln gefüllt, so daß jede Kapsel 5 mg Wirkstoff enthält.

**Beispiel D:** Ampullen

Eine Lösung von 1 kg 5-Methyl-6-[2-(2-pyridyl)-5-benzimidazolyl]-4,5-dihydro-pyridazin-3-on-hydrochlorid-monohydrat in 100 l zweifach destilliertem Wasser wird steril filtriert, in Ampullen abgefüllt, unter sterilen Bedingungen lyophilisiert und steril verschlossen. Jede Ampulle enthält 2 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Patentansprüche**

1. Benzimidazolyl-pyridazinone der Formel I

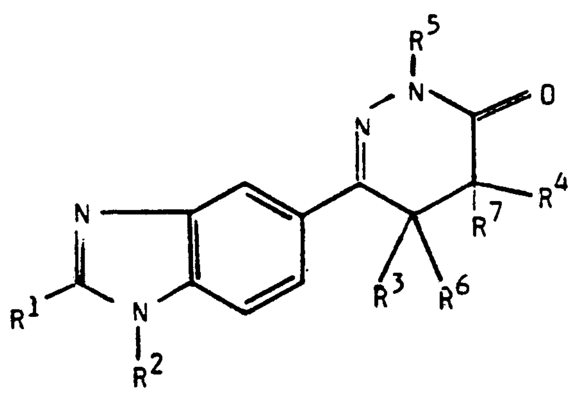

I

worin
$R^1$ einen unsubstituierten oder ein- oder mehrfach durch Alkyl, Alkoxy, Alkylthio, Halogen, OH, SH, Amino, Alkylamino, Dialkylamino, Acylamino mit 1 - 8 C-Atomen, Nitro, COOH, COOAlkyl und/oder CN substituierten Styryl- oder ein- oder zweikernigen, 1 - 4 Heteroatome enthaltenden Heteroarylrest,
$R^2$, $R^3$, $R^4$ und $R^5$ jeweils H oder Alkyl und
$R^6$ und $R^7$ jeweils H oder zusammen eine C-C-Bindung bedeuten und worin die Alkyl- und Alkoxygruppen jeweils 1 - 4 C-Atome enthalten,
sowie deren Salze.

2. a) 5-Methyl-6-[2-(4-pyridyl)-5-benzimidazoly]-4,5-dihydro-pyridazin-3-on;
b) 5-Methyl-6-[2-(3-pyridyl)-5-benzimidazoly]-4,5-dihydro-pyridazin-3-on;
c) 5-Methyl-6-[2-(2-pyridyl)-5-benzimidazoly]-4,5-dihydro-pyridazin-3-on;
d) 5-Methyl-6-[2-(3-thienyl)-5-benzimidazoly]-4,5-dihydro-pyridazin-3-on;
e) 5-Methyl-6-[1-methyl-2-(4-pyridyl)-5-benzimidazoly]-4,5 dihydro-pyridazin-3-on.

3. 5-Methyl-6-[2-(3-pyrazolyl)-5-benzimidazolyl]-4,5-dihydro-pyridazin-3-on.

4. Verfahren zur Herstellung von Benzimidazolyl-pyridazinonen der Formel I sowie ihrer Salze, dadurch gekennzeichnet, daß man ein Diamin der Formel II

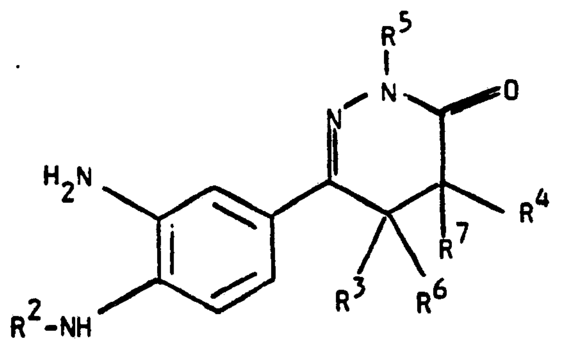

II

worin
$R^2$ $R^3$ $R^4$, $R^5$, $R^6$ und $R^7$ die angegebenen Bedeutungen haben mit einer Säure der Formel $R^1$-COOH (worin $R^1$ die angegebene Bedeutung hat) oder einem ihrer reaktionsfähigen Derivate oder mit einem Aldehyd der Formel $R^1$-CHO (worin $R^1$ die angegebene Bedeutung hat) in Gegenwart eines Oxydationsmittels umsetzt,
oder daß man eine gegebenenfalls im Reaktionsgemisch hergestellte Verbindung der Formel III

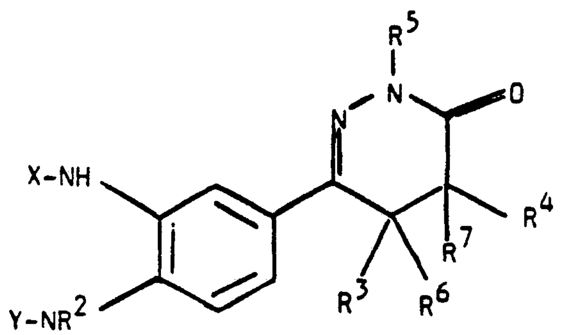

III

worin
einer der Reste X und Y H,
der andere dieser Reste die Gruppe $R^1$-$CZ^1Z^2$-,
$Z^1$ und $Z^2$, die gleich oder verschieden sein können, jeweils gegebenenfalls durch Alkyl substituierte OH- oder SH-Gruppen oder zusammen O, S, NH, N-Alkyl, Alkylendioxy oder Alkylendithio mit jeweils 2 oder 3 C-Atomen bedeuten und
$R^1$ bis $R^7$ die angegebenen Bedeutungen haben cyclisiert
oder daß man eine Ketosäure der Formel IV

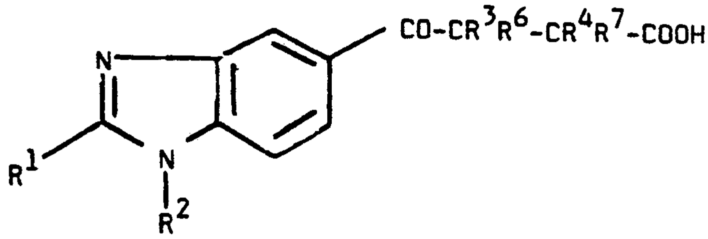

IV

worin

$R^1$, $R^2$, $R^3$, $R^4$, $R^6$ und $R^7$ die angegebenen Bedeutungen haben oder eines ihrer reaktionsfähigen Derivate mit einem Hydrazin der Formel $R^5$-NH-$NH_2$ (worin $R^5$ die angegebene Bedeutung hat) oder einem seiner reaktionsfähigen Derivate umsetzt

und/oder daß man ein Tetrahydropyridazinon der Formel I ($R^6 = R^7 = H$) durch Behandeln mit dehydrierenden Mitteln in das entsprechende Dihydropyridazinon der Formel I ($R^6$ und $R^7$ zusammen = C-C-Bindung) umwandelt

und/oder daß man in einer Verbindung der Formel I eine funktionelle Gruppe in eine andere funktionelle Gruppe umwandelt und daß man gegebenenfalls eine Base der Formel I durch Behandeln mit einer Säure in eines ihrer Salze umwandelt.

5. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Trägeroder Hilfsstoff und gegebenenfalls in Kombination mit einem oder mehreren weiteren Wirkstoff(en) in eine geeignete Dosierungsform bringt.

6. Pharmazeutische Zubereitung, gekennzeichnet durch einen Gehalt an mindestens einer Verbindung der Formel I und/oder einem ihrer physiologisch unbedenklichen Salze.

7. Verbindungen der Formel I zur Bekämpfung von Krankheiten des Herz- und Kreislaufs.

8. Verwendung von Verbindungen der Formel I zur Herstellung eines Arzneimittels.

**Claims**

1. Benzimidazolylpyridazinones of the formula I

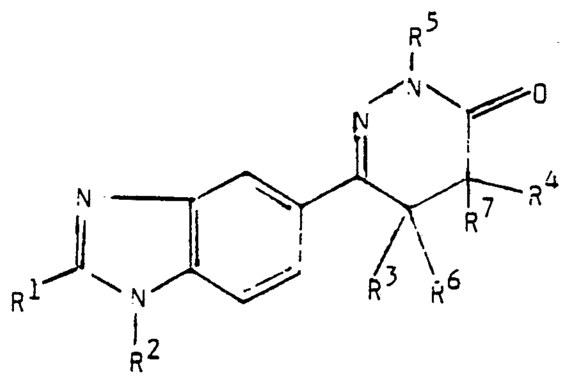

in which

$R^1$ is a styryl or mononuclear or binuclear heteroaryl radical which contains 1-4 heterotoms, each of which is unsubstituted or singly or multiply substituted by alkyl, alkoxy, alkylthio, halogen, OH, SH, amino, alkylamino, dialkylamino, acylamino having 1 - 8 C atoms, nitro, COOH, COOalkyl and/or CN,

$R^2$, $R^3$, $R^4$ and $R^5$ are each H or alkyl, and

$R^6$ and $R^7$ are each H, or together are a C-C bond, and in which the alkyl and alkoxy groups each contain 1 - 4 C atoms,

and their salts.

2. a) 5-Methyl-6-[2-(4-pyridyl)-5-benzimidazolyl]-4,5-dihydro-pyridazin-3-one;
b) 5-Methyl-6-[2-(3-pyridyl)-5-benzimidazolyl]-4,5-dihydro-pyridazin-3-one;
c) 5-Methyl-6-[2-(2-pyridyl)-5-benzimidazolyl]-4,5-dihydro-pyridazin-3-one;
d) 5-Methyl-6-[2-(3-thienyl)-5-benzimidazolyl]-4,5-dihydro-pyridazin-3-one;
e) 5-Methyl-6-[1-methyl-2-(4-pyridyl)-5-benzimidazolyl]-4,5-dihydro-pyridazin-3-one.

3. 5-Methyl-6-[2-(3-pyraxolyl)-5-benzimidazolyl]-4,5-dihydro-pyridazin-3-one.

4. Process for the preparation of benzimidazolylpyridazinones of the formula I, and of their salts, characterised in that a diamine of the formula

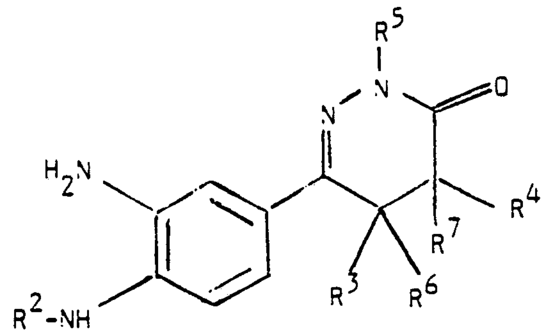

II

in which

$R^2$, $R^3$, $R^4$, $R^5$, $R^6$ and $R^7$ have the indicated meanings, is reacted with an acid of the formula $R^1$-COOH (in which $R^1$ has the indicated meaning) or with one of its reactive derivatives or with an aldehyde of the formula $R^1$ -CHO (in which $R^1$ has the indicated meaning) in the presence of an oxidising agent, or in that a compound of the formula III, which is optionally prepared in the reaction mixture,

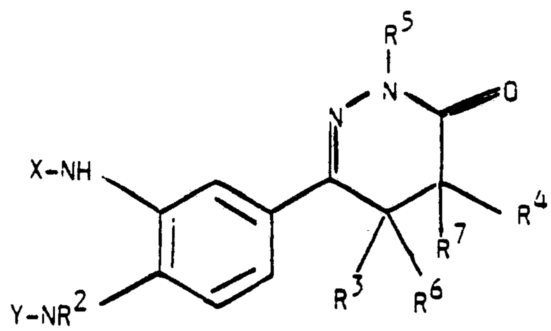

III

in which

one of the radicals X and Y is H,

and the other one of these radicals is the group $R^1CZ^1Z^2$-,

$Z^1$ and $Z^2$, which can be identical or different, are each OH or SH groups which are optionally substituted by alkyl, or together are O, S, NH, N-alkyl, alkylenedioxy or alkylenedithio, each having 2 or 3 C atoms, and

$R^1$ to $R^7$ have the indicated meanings,

is cyclised

or in that a keto acid of the formula IV

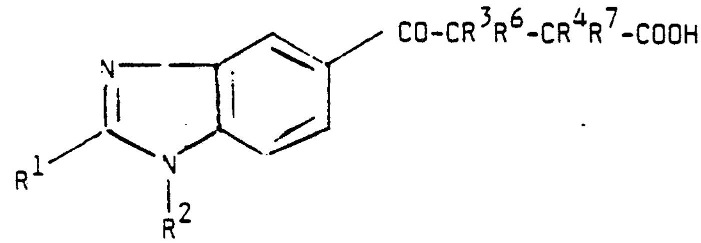

IV

in which

$R^1$, $R^2$, $R^3$, $R^4$, $R^6$ and $R^7$ have the indicated meanings, or one of its reactive derivatives,

is reacted with a hydrazine of the formula $R^5$-NH-$NH_2$ (in which $R^5$ has the indicated meaning) or with one of its reactive derivatives,

and/or in that a tetrahydropyridazinone of the formula ($R^6$ = $R^7$ = H) is converted, by treatment with dehydrogenating agents, into the corresponding dihydropyridazinone of the formula I ($R^6$ and $R^7$ together = C-C bond), and/or in that one functional group in a compound of the formula I is converted into another functional group, and in that, where appropriate, a base of the formula I is converted, by treatment with an acid, into one of its salts.

5. Process for the preparation of pharmaceutical formulations, characterised in that a compound of the formula and/or one of its physiologically acceptable salts is converted into a suitable administration form together with at least one solid, liquid or semi-liquid vehicle or auxiliary and, where appropriate, in combination with one or more other active compound(s).

6. Pharmaceutical formulation characterised by containing at least one compound of the formula I and/or one of its physiologically acceptable salts.

7. Compounds of the formula I for controlling cardiovascular diseases.

8. Use of compounds of the formula I for the preparation of a medicament.

**Revendications**

1. Benzimidazolyl-pyridazinones de formule I:

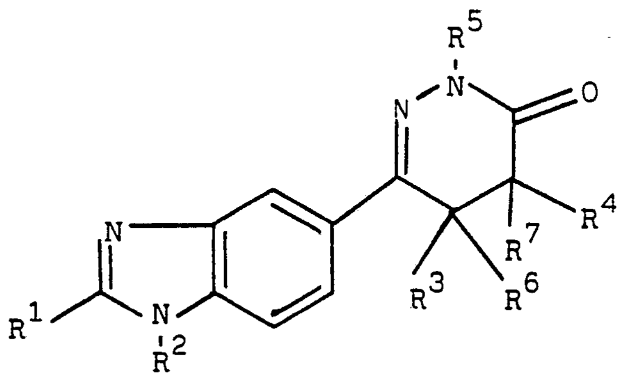

I

dans laquelle

$R^1$ représente un radical hétéro-aryle à un ou deux noyaux, contenant 1 à 4 hétéro-atomes ou un radical styryle non substitué ou substitué une ou plusieurs fois par un groupe alkyle, un groupe alcoxy, un groupe alkylthio, un atome d'halogène, un groupe OH, un groupe SH, un groupe amino, un groupe alkylamino, un groupe dialkylamino, un groupe acylamino contenant 1 à 8 atomes de carbone, un groupe nitro, un groupe COOH, un groupe COOAlkyle et/ou par un groupe CN,

$R^2$, $R^3$, $R^4$ et $R^5$ représentent chacun H ou un groupe alkyle, et

$R^6$ et $R^7$ représentent chacun H ou, ensemble, ils représentent une liaison C-C, les groupes alkyle et alcoxy contenant chacun 1 à 4 atomes de carbone,

ainsi que leurs sels.

2. a) la 5-méthyl-6-[-2-(4-pyridyl)-5-benzimidazolyl-]-4,5-dihydro-pyridazin-3-one;
b) la 5-méthyl-6-[-2-(3-pyridyl)-5-benzimidazolyl]-4,5-dihydro-pyridazin-3-one;
c) la 5-méthyl-6-[-2-(2-pyridyl)-5-benzimidazolyl]-4,5-dihydro-pyridazin-3-one;
d) la 5-méthyl-6-[-2-(3-thiényl)-5-benzimidazolyl]-4,5-dihydro-pyridazin-3-one;
e) la 5-méthyl-6-[1-méthyl-2-(4-pyridyl)-5-benzimidazolyl]-4,5-dihydro-pyridazin-3-one.

3. La 5-méthyl-6-[ 2-(3-pyrazolyl)-5-benzimidazolyl]-4,5-dihydro-pyridazin-3-one.

4. Procédé de préparation de benzimidazolyl-pyridazinones de formule I, ainsi que de leurs sels, caractérisé en ce qu'on fait réagir une diamine de formule II:

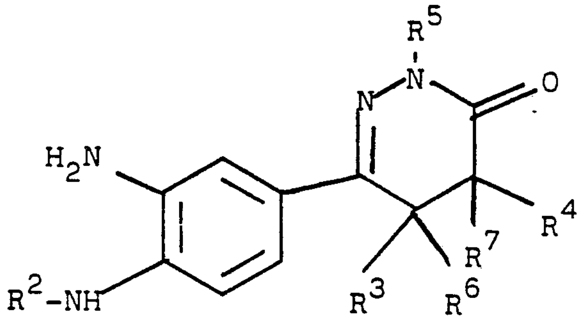

II

dans laquelle

$R^2$, $R^3$, $R^4$, $R^5$, $R^6$ et $R^7$ ont les significations indiquées, avec un acide de formule $R^1$-COOH (dans laquelle $R^1$ a la signification indiquée) ou un de ses dérivés réactifs ou encore avec un aldéhyde de formule $R^1$-CHO (dans laquelle $R^1$ a la signification indiquée), en présence d'un agent d'oxydation, ou en ce qu'on cyclise un composé (éventuellement préparé dans le mélange réactionnel) répondant à la formule III:

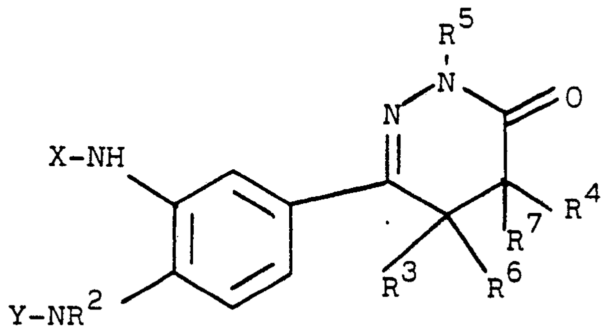

III

dans laquelle

un des radicaux X et Y représente H, tandis que l'autre radical représente le groupe $R^1$-$CZ^1Z^2$-,

$Z^1$ et $Z^2$ peuvent être identiques ou différents et représentent chacun un groupe OH ou SH éventuellement substitué par un groupe alkyle ou, ensemble, ils représentent O, S, NH, un groupe N-alkyle, un groupe alkylène-dioxy ou un groupe alkylène-dithio contenant chacun 2 ou 3 atomes de carbone, et

$R^1$ à $R^7$ ont les significations indiquées, ou cn fait réagir un céto-acide de formule IV:

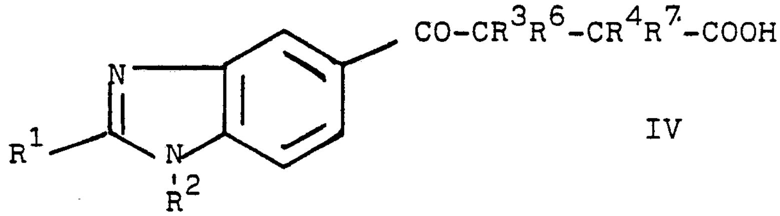

IV

dans laquelle

$R^1$, $R^2$, $R^3$, $R^4$, $R^6$ et $R^7$ ont les significations indiquées ou un de ses dérivés réactifs, avec une hydrazine de formule $R^5$-NH-$NH_2$ (dans laquelle $R^5$ a la signification indiquée) ou un de ses dérivés réactifs,

et/ou transforme une tétrahydropyridazinone de formule I ($R^6$ = $R^7$ = H) en dihydropyridazinone correspondante de formule I ($R^6$ et $R^7$ ensemble = liaison C-C) par traitement avec des agents de déshydrogénation,

et/ou, dans un compose de formule I, on transforme un groupe fonctionnel en un autre groupe fonctionnel,

et on transforme éventuellement une base de formule I en un de ses sels par traitement avec un acide.

5. Procédé en vue d'obtenir des préparations pharmaceutiques, caractérisé en ce qu'on met, sous une forme posologique appropriée, un composé de formule I et/ou un de ses sels physiologiquement inoffensifs conjointement avec au moins une substance auxiliaire ou une substance support solide, liquide ou semi-liquide et éventuellement en combinaison avec une ou plusieurs autres substances actives.

6. Préparation pharmaceutique, caractérisée en ce qu'elle contient au moins un composé de formule I et/ou un de ses sels physiologiquement inoffensifs.

7. Composés de formule I en vue de combattre les maladies du coeur et de la circulation.

8. Utilisation de composés de formule I pour la préparation d'un médicament.